(19) Europäisches Patentamt
European Patent Office
Office européen des brevets

(11) EP 0 796 840 A2

(12) **EUROPÄISCHE PATENTANMELDUNG**

(43) Veröffentlichungstag:
**24.09.1997 Patentblatt 1997/39**

(21) Anmeldenummer: **97810144.2**

(22) Anmeldetag: **13.03.1997**

(51) Int Cl.⁶: **C07C 219/06**, C07C 233/36, C07C 233/56, C07C 233/62, C07C 235/60, C07D 251/34, C07D 251/70, C08K 5/00, C08L 57/08

(84) Benannte Vertragsstaaten:
**AT BE DE DK ES FR GB IT NL SE**

(30) Priorität: **22.03.1996 CH 753/96**

(71) Anmelder: **Ciba Specialty Chemicals Holding Inc.**
**4057 Basel (CH)**

(72) Erfinder:
- **Köstler, Hans-Günter**
  **64646 Heppenheim (DE)**
- **Kuhn, Karl Josef**
  **64686 Lautertal (DE)**
- **Wehner, Wolfgang**
  **64372 Ober-Ramstadt (DE)**
- **Zinke, Horst**
  **64385 Reichelsheim/Odw. (DE)**

(54) **Stabilisatoren für chlorhaltige Polymere**

(57) Es werden Stabilisatoren der Formeln I und II für in der Hauptsache PVC beschrieben, die besonders gut mit Zinkcarboxylat kombiniert werden können:

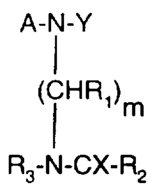

**(I)** ,

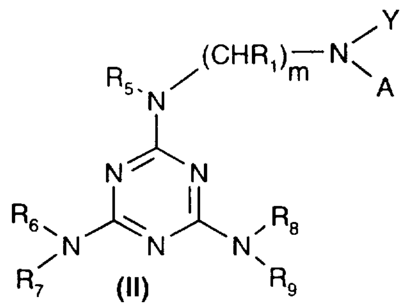

**(II)**

worin die Reste A, X, Y und $R_1$ bis $R_9$ sowie m wie in Anspruch 1 definiert sind.

EP 0 796 840 A2

**Beschreibung**

Die Erfindung betrifft Amin-Verbindungen der unten dargestellten Formeln I und II, die sich zum Stabilisieren von chlorhaltigen Polymeren, besonders PVC, eignen.

PVC kann durch eine Reihe von Zusatzstoffen stabilisiert werden. Verbindungen des Bleis, Bariums und Cadmiums sind dafür besonders gut geeignet, sind jedoch heute aus ökologischen Gründen umstritten. (vgl. "Kunststoffadditive", Herausgeber R. Gächter und H. Müller, Carl Hanser Verlag, 3.Aufl. 1989, Seiten 303-311, und "Kunststoff Handbuch PVC", 2. Aufl. 1985, Band 2/1, W. Becker/D. Braun, Carl Hanser Verlag 1985, Seiten 531-538; sowie Kirk-Othmer: Encyclopedia of Chemical Technology 4th ed. 1994, Vol 12, "Heat Stabilizers", S. 1071-1091). Man sucht daher weiter nach wirksamen Stabilisatoren und Stabilisatorkombinationen.

Es wurden nun neue Stabilisatoren gefunden, die für die Stabilisierung von chlorhaltigen Polymeren, insbesondere PVC, geeignet sind.

Die vorliegende Erfindung betrifft daher Verbindungen der Formel I und II

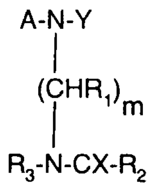
(I) ,

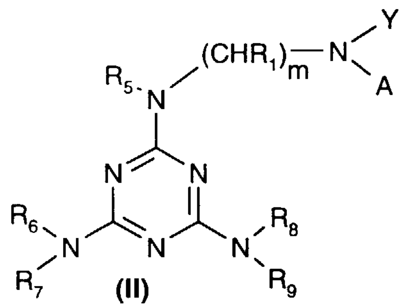
(II)

worin A und Y unabhängig voneinander $C_1$-$C_8$ -Alkyl, $C_3$-$C_8$-Alkenyl, $C_5$-$C_8$-Cycloalkyl oder $C_7$-$C_9$-PhenyPaekyl sind oder zusammen gegebenenfalls durch O, NH oder N-$CH_3$ unterbrochenes $C_2$-$C_5$-Alkylen bilden,

$R_1$: H oder $C_1$-$C_4$-Alkyl,
$R_2$ : $C_1$-$C_8$-Alkyl, $NH_2$ ,

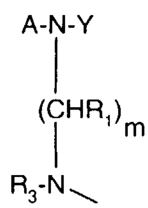

, $R_4$-O,

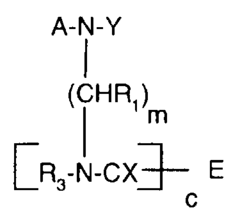
,

Phenyl oder mit OH, $C_1$-$C_4$-Alkyl-O, $C_1$-$C_4$-Alkyl substituiertes Phenyl,
$R_3$: H, $C_1$-$C_8$-Alkyl, $C_3$-$C_8$-Alkenyl, $C_5$-$C_8$-Cycloalkyl,

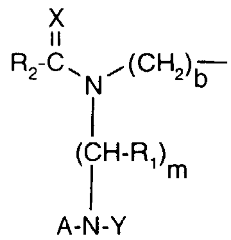

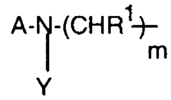 ,

,

$R_4$ : $C_1$-$C_4$-Alkyl, Cyclohexyl, Benzyl;

E : $-(CH_2)_a-$, -CH=CH-,

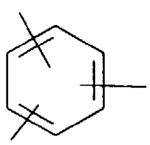 ,

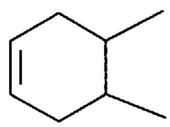 ,

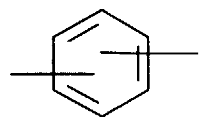 ,

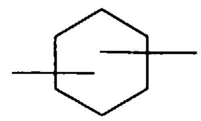 ,

-CH(OH)-CH(OH)-, -NH $(CH_2)_b$NH-;

X: O oder S;

a: 0 bis 8; b: 2 bis 12; c: 1 oder 2; m: 2 bis 6, und

$R_5$ bis $R_9$ unabhängig voneinander H, $C_1$-$C_8$ -Alkyl, $C_3$-$C_8$-Alkenyl, $C_5$-$C_8$-Cycloalkyl, $C_7$-$C_9$-Phenylalkyl, HO-$C_2H_4$-, HO-$C_3H_6$-,

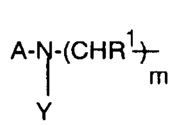 ,

oder $R_6$ und $R_7$ bzw. $R_8$ und $R_9$ jeweils zusammen gegebenenfalls durch O, NH oder N-$CH_3$ unterbrochenes $C_2$-$C_5$-Alkylen bilden.

Bevorzugt betrifft die Erfindung eine Stabilisatorkombination enthaltend

**A)** mindestens eine organischen Zinkverbindung mit einer Zn-O- oder Zn-S-Bindung, insbesondere ein Zinkcarboxylat, und

**B)** mindestens eine Verbindung der Formel I oder II,

sowie deren Verwendung zum Stabilisieren von chlorhaltigen Polymeren, besonders PVC.

Bevorzugt sind (gegebenenfalls in der Stabilisatorkombination) Verbindungen der Formeln I und II, worin

A und Y unabhängig voneinander $C_1$-$C_8$ -Alkyl oder $C_5$-$C_8$-Cydoalkyl sind oder zusammen gegebenenfalls durch O, NH oder N-$CH_3$ unterbrochenes $C_2$-$C_5$ -Alkylen bilden,

$R_1$: H,

$R_2$ : $C_1$-$C_8$-Alkyl, $NH_2$,

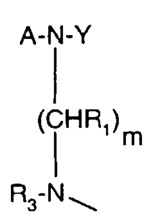 ,

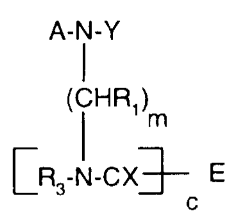 ,

Phenyl,

$R_3$: H, $C_1$-$C_8$-Alkyl,

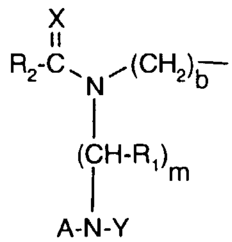

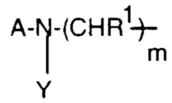 ,

,

E : -$(CH_2)_a$-, -CH=CH-,

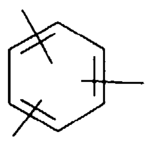 , 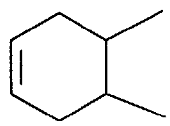 , 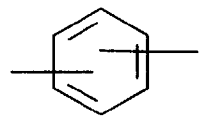

,

-NH $(CH_2)_b$NH-;

X: O,

a: 0 bis 4; b: 2 bis 6; c: 1 oder 2, m: 2 oder 3 sind, und

$R_5$ bis $R_9$ unabhängig voneinander H, $C_1$-$C_8$ -Alkyl, $C_5$-$C_8$-Cycloalkyl, HO-$C_2H_4$-, HO-$C_3H_6$-,

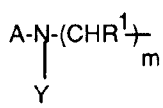 ,

oder $R_6$ und $R_7$ bzw. $R_8$ und $R_9$ jeweils zusammen gegebenenfalls durch O, NH oder N-$CH_3$ unterbrochenes $C_2$-$C_5$-Alkylen bilden.

Besonders bevorzugt sind Verbindungen der Formeln I und II, worin A und Y gleich und $C_1$-$C_4$ -Alkyl oder Cyclohexyl sind,

$R_1$: H,

$R_2$:

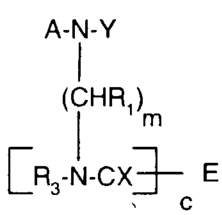

,

$R_3$: H, $C_1$-$C_4$-Alkyl,

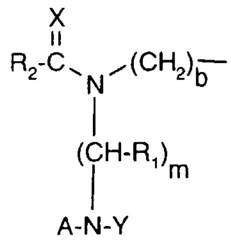

oder

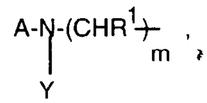

E : $-(CH_2)_a-$, oder $-NH\ (CH_2)_bNH-$;
X: O ;
a: 0 bis 4; b: 2 bis 6; c: 1 oder 2; m: 2 oder 3; und
$R_5$ bis $R_9$ unabhängig voneinander H, $C_1$-$C_4$ -Alkyl oder

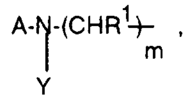

sind.

In obigen Formeln bedeuten:

$C_1$-$C_4$-Alkyl: Methyl, Ethyl, n-Propyl, iso-Propyl, n-, i-, sec- oder t-Butyl;
$C_1$-$C_8$-Alkyl: dieselben Reste und z.B. n-Pentyl, Isopentyl, Neopentyl, n-Hexyl, n-Heptyl, 2-Ethylhexyl, n-Octyl oder iso-Octyl;
$C_5$-$C_8$-Cycloalkyl: z.B. Cyclopentyl, Cyclohexyl, Cycloheptyl, oder Cyclooctyl. Cyclopentyl und insbesondere Cyclohexyl sind bevorzugt;
$C_3$-$C_8$-Alkenyl: verzweigtes oder unverzweigtes Alkenyl wie z.B. Allyl, 2-Methylallyl oder Hexenyl;
$C_1$-$C_4$-Alkoxy: z.B. Methoxy, Ethoxy, Propoxy, Isopropoxy, Butoxy oder Isobutoxy;
$C_7$-$C_9$-Phenylalkyl: beispielsweise Benzyl, 1- oder 2-Phenylethyl, 3-Phenylpropyl, α,α-Dimethylbenzyl oder 2-Phenylisopropyl, vorzugsweise Benzyl.

Beispiele für Verbindungen der Formel I sind:

1) 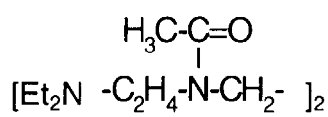

2) $[^{iso}Pr_2N-CH_2CH_2-NH-CO-CH_2CH_2-]_2$

3) $[^{iso}Pr_2N-CH_2CH_2CH_2-NH-CO-]_2$

4) 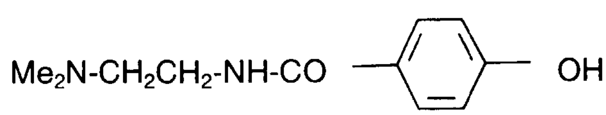

5) 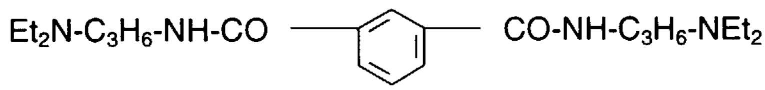

6) 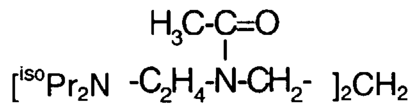

7) 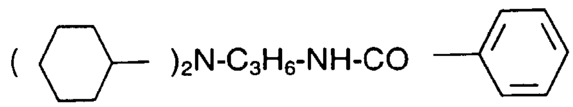

8) 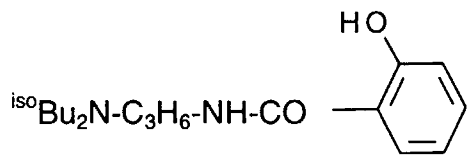

9) $(^{tert}Bu_2N\text{-}C_3H_6\text{-}NH)_2CO$

10) 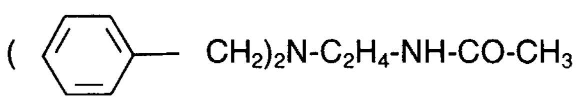

11) $(Et_2N\text{-}C_2H_4\text{-}NH\text{-}CO\text{-})_2$

12) 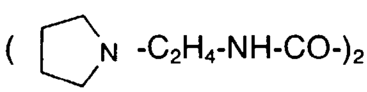

13) 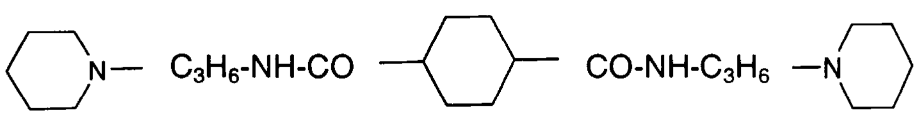

14) $[^{iso}Pr_2N\text{-}C_2H_4\text{-}NH\text{-}CO\text{-}CH(OH)]_2$

15) $(Et_2N\text{-}C_3H_6\text{-}N(CH_3)\text{-}CO\text{-}CH_2\text{-})_2$

16) $(^{iso}Pr_2N\text{-}C_3H_6\text{-})_2N\text{-}CO\text{-}CH_3$

17) {[( 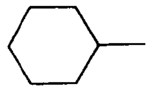 $)_2N\text{-}C_2H_4\text{-}]_2N\text{-}CO\text{-}CH_2\}_2$

18) $(^{n}Pr_2N\text{-}C_3H_6)_2N\text{-}CO$ 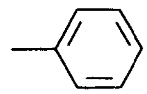

19) ( 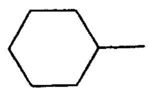 $)_2N\text{-}C_3H_6NBu\text{-}CO\text{-}O\text{-}Bu$

20) $Bu_2N\text{-}CH_2CH_2\text{-}NAc\text{-}CH_2$ 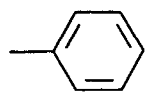

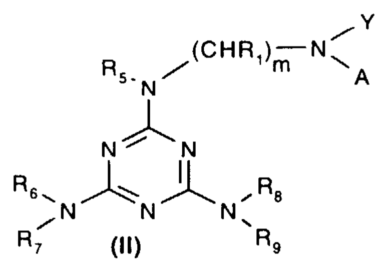

(II)

Beispiele für Verbindungen der Formel II sind:

| | $AYN-(CHR_1)_m-NR_5-$ | $R_6R_7N-$ | $R_8R_9N-$ |
|---|---|---|---|
| 21) | $Et_2N-C_2H_4-NH-$ | $-NH_2$ | $-NH_2$ |
| 22) | $Et_2N-C_2H_4-NH-$ | $Et_2N-C_2H_4-NH-$ | $Et_2N-C_2H_4-NH-$ |
| 23) | $^{n}Pr_2N-C_3H_6-NH-$ | $HO-C_2H_4-NH-$ | $HO-C_2H_4-NH-$ |
| 24) | $^{iso}Pr_2N-C_2H_4-NH-$ | $(HO-C_2H_4-)_2N-$ | $(HO-C_2H_4-)_2N-$ |
| 25) | ( 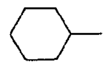 $)_2N-C_2H_4-NH-$ | $Et-NH-$ | ( 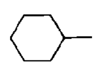 $)_2N-C_2H_4-$ NH- |
| 26) | 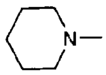 $C_3H_6-NH-$ | 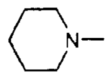 $C_3H_6-NH-$ | Me 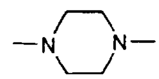 |
| 27) | 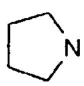 $-C_2H_4-NH-$ | 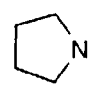 - | 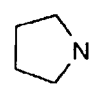 - |
| 28) | 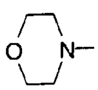 $C_3H_6-NH-$ | 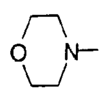 $C_3H_6-NH-$ | 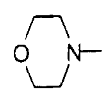 |
| 29) | $^{iso}Pr_2N-C_2H_4-NH-$ | $^{iso}Pr_2N-C_2H_4-NH-$ | $^{iso}Pr_2N-$ |
| 30) | $^{iso}Pr_2N-C_2H_4-NH-$ | $^{iso}Pr_2N-C_2H_4-NH-$ | $^{iso}PrNEt-$ |
| 31) | $Et_2N-C_2H_4-NH-$ | $Et_2N-C_2H_4-NH-$ | ( 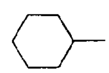 $)_2N-$ |

Dabei sind: Me = Methyl, Et = Ethyl, Bu = Butyl, $^{tert}Bu$ = tertiär-Butyl, $^{iso}Pr$ = Isopropyl, $^{n}Pr$ = normal-Propyl, Ac = Acetyl.

Die Verbindungen der Komponente A) werden zur Stabilisierung im chlorhaltigen Polymer zweckmäßig zu 0,01 bis 10, vorzugsweise zu 0,05 bis 5, insbesondere zu 0,1 bis 3 Teilen auf 100 Teile Polymer eingesetzt.

**Zinkverbindungen:** Bei den organischen Zinkverbindungen mit einer Zn-O-Bindung handelt es sich um Zinkenolate, Zinkphenolate oder/und Zinkcarboxylate. Letztere sind Verbindungen aus der Reihe der aliphatischen gesättigten und ungesättigten $C_{2}$-$_{22}$-Carboxyratey der aliphatischen gesättigten oder ungesättigten $C_{2}$-$_{22}$-Carboxylate, die mit wenigstens einer OH-Gruppe substituiert sind oder deren Kette wenigstens durch ein oder mehrere O-Atome unterbrochen ist (Oxasäuren), der cyclischen und bicyclischen Carboxylate mit 5-22 C-Atomen, der unsubstituierten, mit wenigstens einer OH-Gruppe substituierten und/oder $C_{1}$-$_{16}$-alkylsub-stituierten Phenylcarboxylate, der Phenyl-$C_{1-16}$-alkylcarboxylate, oder der gegebenenfalls mit $C_{1-12}$-Alkyl substituierten Phenolate, oder der Abietinsäure.

Zn-S-Verbindungen sind beispielsweise Zn-Mercaptide, Zn-Mercaptocarboxylate und Zn-Mercaptocarbonsäureester.

Namentlich zu erwähnen sind, als Beispiele, die Zinksalze der monovalenten Carbonsäuren, wie Essigsäure, Propionsäure, Buttersäure, Valeriansäure, Hexansäure, Önanthsäure, Octansäure, Neodecansäure, 2-Ethylhexansäure, Pelargonsäure, Decansäure, Undecansäure, Dodecansäure, Tridecansäure, Myristylsäure, Palmitinsäure, Laurylsäure, Isostearinsäure, Stearinsäure, 12-Hydroxystearinsäure, 9,10-Dihydroxystearinsäure, Ölsäure, Ricinolsäure, 3,6-Dioxaheptansäure, 3,6,9-Trioxadecansäure, Behensäure, Benzoesäure, p-tert-Butyl-benzoesäure, Dimethylhydroxybenzoesäure, 3,5-Di-tert-butyl-4-hyd roxy-benzoesäure, Tolylsäure, Dimethylbenzoesäure,

Ethylbenzoesäure, n-Propylbenzoesäure, Salicylsäure, p-tert-Octylsalicylsäure, und Sorbinsäure, Zimtsäure, Mandelsäure, Glykolsäure; Zinksalze der divalenten Carbonsäuren bzw. deren Monoester, wie Oxalsäure, Malonsäure, Bernsteinsäure, Glutarsäure, Adipinsäure, Fumarsäure, Pentan-1,5-dicarbonsäure, Hexan-1,6-dicarbonsäure, Heptan-1 ,7-dicarbonsäure, Octan-1 ,8-dicarbonsäure, 3,6,9-Trioxadecan-1,10-dicarbonsäure, Milchsäure, Malonsäure, Maleinsäure, Weinsäure, Äpfelsäure, Salicylsäure, Polyglykol-dicarbonsäure (n=10-12), Phthalsäure, Isophthalsäure, Terephthalsäure und Hydroxyphthalsäure; und der Di- oder Triester der tri-oder tetravalenten Carbonsäuren, wie Hemimellithsäure, Trimellithsäure,
Pyromellithsäure, Zitronensäure sowie ferner sog. überbasische (overbased) Zinkcarboxylate oder Zinklaurylmercaptid, Zinkthioglykolat, Zinkthiosalicylat, Zink-bis-i-octylthioglykolat, Zinkmercaptopropionat, Zinkthiolactat, Zinkthiomalat, Zink-bis-octylmercaptopropionat, Zink-bis-isooctylthiolactat und Zink-bis-laurylthiomalat.

Bei den Zinkenolaten handelt es sich bevorzugt um Enolate des Acetylacetons, des Benzoylacetons, des Dibenzoylmethans sowie um Enolate der Acetessig- und Benzoylessigester sowie der Dehydracetsäure. Außerdem können auch anorganische Zinkverbindungen wie Zinkoxid, Zinkhydroxid, Zinkcarbonat, basisches Zinkcarbonat oder Zinksulfid zum Einsatz kommen.

Bevorzugt sind neutrale oder basische Zinkcarboxylate einer Carbonsäure mit 2 bis 22 C-Atomen (Zinkseifen), wie beispielsweise Benzoate oder Alkanoate, bevorzugt C8-Alkanoate, Stearat, Oleat, Laurat, Palmitat, Behenat, Versatat, Hydroxystearate und -oleate, Dihydroxystearate, p-tert-Butylbenzoat, oder (Iso)octanoat. Besonders bevorzugt sind Stearat, Oleat, Versatat, Benzoat, p-tert-Butylbenzoat und 2-Ethylhexanoat.

Neben den genannten Zink-Verbindungen kommen auch (an)organische Aluminium-Verbindungen in Frage, die eine Al-O-Bindung aufweisen. Zu den verwendbaren und bevorzugten Aluminium-Verbindungen gehören u.a. bevorzugt Aluminiumenolate und Aluminiumcarboxylate. Beispiele für Carboxylat- und Enolatreste sind den oben unter Zink aufgeführten entsprechend zu entnehmen.

Beispiele für anorganische Al-Verbindungen sind Aluminiumhydroxid und Aluminiumphosphate.

Die beschriebenen Metallseifen bzw. deren Mischungen können in Mengen von beispielsweise 0,001 bis 10, zweckmäßig 0,01 bis 5, bevorzugt 0,01 bis 3 Gew.-Teilen, bezogen auf 100 Gew.-Teile chlorhaltiges Polymer, angewandt werden.

Sie können auch als Mischsalze (Copräzipitate) vorliegen.

Die erfindungsgemäße Stabilisatorkombination kann zusammen mit weiteren Zusätzen verwandt werden, die für die Verarbeitung und Stabilisierung chlorhaltiger Polymere üblich sind, wie z.B.

1. Stabilisatoren: Epoxide und epoxidierte Fettsäureester; Phosphite; Thiophosphite und Thiophosphate; Polyole; 1,3-Dicarbonylverbindungen; Mercaptocarbonsäureester; Dihydropyridine; Antioxidantien; Lichtschutzmittel und UV-Absorber; Alkali- und Erdalkaliverbindungen; Perchlorat-Salze; Zeolithe; Hydrotalcite; Dawsonite;

2. weitere gängige PVC-Zusatzstoffe wie z.B. Gleitmittel; Weichmacher; Schlagzähigkeitsmodifikatoren; Verarbeitungshilfen; Treibmittel; Füllstoffe; Antistatika; Biocide; Antifogging-Agents; Pigmente und Farbstoffe; Metalldesaktivatoren; Flammschutzmittel (Vgl. dazu "Handbook of PVC-Formulating" von E. J. Wickson, John Wiley & Sons, New York 1993).

Beispiele für solche Zusatzstoffe sind dem Fachmann bekannt und in der Fachliteratur zu finden. Nicht beschränkend seien hier einige der bekannten Zusätze und Verarbeitungshilfsmittel genannt:

**Phosphite:** Organische Phosphite sind bekannte Co-Stabilisatoren für chlorhaltige Polymere. Beispiele sind Trioctyl-, Tridecyl-, Tridodecyl-, Tritridecyl-, Tripentadecyl-, Trioleyl, Tristearyl-, Triphenyl-, Trikresyl-, Tris-nonylphenyl- Tris-2,4-t-butyl-phenyl- oder Tricyclohexylphosphit.

Weitere geeignete Phosphite sind verschieden gemischte Aryl-dialkyl. bzw Alkyldiarylphosphite wie Phenyldioctyl-, Phenyldidecyl-, Phenyldidodecyl-, Phenylditridecyl-, Phenylditetradecyl-, Phenyldipentadecyl-, Octyldiphenyl-, Decyldiphenyl-, Undecyldiphenyl-, Dodecyldiphenyl-, Tridecyldiphenyl-, Tetradecyldiphenyl-, Pentadecyldiphenyl-,Oleyldiphenyl-, Stearyldiphenyl- und Dodecyl-bis-2,4-di-t-butylphenylphosphit.

Weiterhin können auch Phosphite verschiedener Di- bzw. Polyole vorteilhaft verwandt werden: z.B. Tetraphenyldipropylenglykoldiphosphit, Polydipropylen-glykolphenylphosphit, Tetramethylolcyclohexanol-decyldiphosphit, Tetramethylol-cyclohexanol-butoxyethoxy-ethyldiphosphit, Tetramethylolcyclohexanol-nonylphenyldiphosphit, Bis-nonylphenyl-di-trimethylolpropandiphosphit, Bis-2-butoxyethyl-di-trimethylolpropandiphosphit, Trishydroxyethylisocyanurat-hexadecyltriphosphit, Didecylpentaerythritdiphosphit, Distearylpentaerythrit-diphosphit, Bis-2,4-di-t-butylphenylpentaerythritdiphosphit, sowie Gemische dieser Phosphite und Aryl/alkylphosphit-Gemische der statistischen Zusammensetzung $(H_{19}C_9\text{-}C_6H_4)O_{1,5}P(OC_{12,13}H_{25,27})_{1,5}$ oder $[C_8H_{17}\text{-}C_6H_4\text{-}O\text{-}]_2P[i\text{-}C_8H_{17}O]$ oder $(H_{19}C_9\text{-}C_6H_4)O_{1,5}P(OC_{9,11}H_{19,23})_{1,5}$

Die organischen Phosphite können in einer Menge von beispielsweise 0,01 bis 10, zweckmäßig 0,05 bis 5

und insbesondere 0,1 bis 3 Gew.-Teilen, bezogen auf 100 Gew.-Teile PVC, angewandt werden.

**Polyole:** Als Verbindungen dieses Typs kommen beispielsweise in Betracht:

Pentaerythrit, Dipentaerythrit, Tripentaerythrit, Bistrimethylolpropan, Trimethylolethan, Bistrimethylolethan, Trimethylolpropan, Sorbit, Maltit, Isomaltit, Lactit, Lycasin, Mannit, Lactose, Leucrose, Tris-(hydroxyethyl)-isocyanurat, Palatinit, Tetramethylolcyclohexanol (TMCH), Tetramethylolcyclopentanol, Tetramethylol-cyclopyranol, Glycerin, Diglycerin, Polyglycerin, Thiodiglycerin, oder 1-0-α-D-Glyco-pyranosyl-D-mannit-dihydrat sowie Polyvinylalkohol und Cyclodextrine. Bevorzugt sind davon TMCH und die Disaccharidalkohole.

Die Polyole können in einer Menge von beispielsweise 0,01 bis 20, zweckmäßig 0,1 bis 20 und insbesondere 0,1 bis 10 Gew.-Teilen, bezogen auf 100 Gew.-Teile PVC, angewandt werden.

**1,3-Dicarbonylverbindungen:** Beispiele für 1,3-Dicarbonylverbindungen sind Acetylaceton, Butanoylaceton, Heptanoylaceton, Stearoylaceton, Palmitoylaceton, Lauroylaceton, 7-tert.Nonylthio-heptandion-2,4, Benzoylaceton, Dibenzoylmethan, Lau roylbenzoylmethan, Palmitoyl-benzoylmethan, Stearoyl-benzoylmethan, Isooctylbenzoylmethan, 5-Hydroxycapronyl-benzoylmethan, Tribenzoylmethan, Bis(4-methylbenzoyl)methan, Benzoyl-p-chlorbenzoylmethan, Bis(2-hydroxy-benzoyl)methan, 4-Methoxybenzoyl-benzoylmethan, Bis(4-methoxybenzoyl) methan, 1-Benzoyl-1-acetylnonan, Benzoyl-acetyl-phenylmethan, Stearoyl-4-methoxy-benzoylmethan, Bis(4-tert-butylbenzoyl)methan, Benzoyl-formylmethan, Benzoyl-phenylacetylmethan, Bis(cyclohexanoyl)methan, Di(pivaloyl)methan, Acetessig-säure-methylester, -ethylester, -hexylester, -octylester, -dodecylester oder - octadecylester, Benzoylessigsäure-ethylester, -butylester, -2-ethylhexylester, - dodecylester oder -octadecylester, Stearoylessigsäure-ethyl-, -propyl-, -butyl-, - hexyl- oder -octylester und Dehydracetsäure sowie deren Zink-, Alkali, Erdalkali- oder Aluminiumsalze.

Die 1,3-Dicarbonylverbindungen können in einer Menge von beispielsweise 0,01 bis 10, zweckmäßig 0,01 bis 3 und insbesondere 0,01 bis 2 Gew.-Teilen, bezogen auf 100 Gew.-Teile PVC, angewandt werden.

**Thiophosphite und Thiophosphate:** Unter Thiophosphiten bzw.
Thiophosphaten sind Verbindungen vom allgemeinen Typ
$(RS)_3P$, $(RS)_3P=O$ bzw. $(RS)_3P=S$ zu verstehen, wie sie in den Patentschriften DE 2809492, EP 090770 und EP 573394 beschrieben sind. Beispiele sind: Trithiohexylphosphit, Trithiooctylphophit, Trithiolaurylphosphit, Trithiobenzylphosphit, Trithiophosphorigsäure-tris-[carboxy-i-octyloxy]-methylester, Trithiophosphorsäure-S,S,S-tris-[carbo-i-octyloxy]-methylester, Trithiophosphorsäure-S,S,S-tris-[carbo-2-ethylhexyloxy]-methylester, Trithiophosphorsäure-S,S,S,-tris-1-[carbo-hexyloxy]-ethylester, Trithiophosphorsäure-S,S,S-tris-1-[carbo-2-ethylhexyloxy]-ethylester, Trithiophosphorsäure-S,S,S-tris-2-[carbo-2-ethylhexyloxy]-ethylester.

Die Thiophosphite bzw. Thiophosphate können zweckmäßig zu 0,01 bis 20, bevorzugt zu 0,1 bis 5, insbesondere zu 0,1 bis 1 % im chlorhaltigen Polymer vorliegen.

**Mercaptocarbonsäureester:** Beispiele für diese Verbindungen sind: Ester der Thioglykolsäure, Thioäpfelsäure, Mercaptopropionsäure, der Mercaptobenzoesäuren bzw. der Thiomilchsäure, wie sie in FR 2459816, EP 90748, FR 2552440 und EP 365483 beschrieben sind. Die Mercaptocarbonsäureester umfassen auch entsprechende Polyolester bzw. deren Partialester.

Sie können zweckmäßig zu 0,01 bis 10, bevorzugt zu 0,1 bis 5, insbesondere zu 0,1 bis 1 % im chlorhaltigen Polymer vorliegen.

**Epoxide und epoxidierte Fettsäureester:** Die erfindungsgemäße Stabilisatorkombination kann zusätzlich mindestens einen epoxidierten Fettsäureester enthalten. Es kommen dafür vor allem Ester von Fettsäuren aus natürlichen Quellen, wie Sojaöl oder Rapsöl, in Frage.

Die Epoxyverbindungen werden in Mengen von beispielsweise ab 0,1 Teil, bezogen auf 100 Gew.-Teile Zusammensetzung, zweckmäßig von 0,1 bis 30, vorzugsweise von 0,5 bis zu 25 Gew.-Teilen, angewandt. Weitere Beispiele sind epoxidiertes Polybutadien, epoxidiertes Leinsamenöl, epoxidiertes Fischöl, epoxidierter Talg, Methylbutyl- oder 2-Ethylhexylepoxystearat, Tris(epoxypropyl)isocyanurat, epoxidiertes Ricinusöl, epoxidiertes Sonnenblumenöl, 3-Phenoxy-1,2-epoxypropan, Bisphenol-A-diglycidylether, Vinylcyclohexendiepoxyd, Dicyclopentadiendiepoxyd und 3,4-Epoxycyclohexylmethyl-3,4-epoxycyclohexancarboxylat.

Als **Epoxide** kommen auch Bisphenol-A- und Bisphenol-F-derivate in Frage wie z.B. in der südafrikanischen Patentschrift ZA-2600/94 beschrieben.

**Dihydropyridine und Polydihydropyridine:** Als monomere Dihydropyridine kommen Verbindungen, wie z. B. in FR 2039496, EP 2007 , EP 362012 und EP 24754 beschrieben, in Frage. Bevorzugt sind solche der Formel

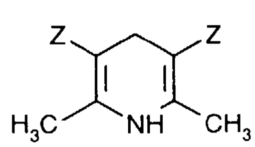

, worin Z für $CO_2CH_3$, $CO_2C_2H_5$, $CO_2{}^nC_{12}H_{25}$ oder $-CO_2C_2H_4-S-{}^nC_{12}H_{25}$ steht.
Als Polydihydropyridine kommen vor allem Verbindungen der folgenden Formel in Frage

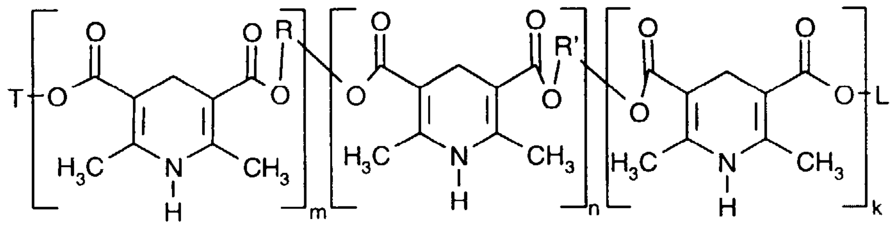

worin T für unsubstituiertes $C_1$-$_{12}$ Alkyl steht,

L dieselben Bedeutungen wie T hat,
m und n Zahlen von 0 bis 20 bedeuten, k 0 oder 1 ist, p 2 bis 8 ist ,
t 0 bis 10 ist
R und R unabhängig voneinander Aethylen, Propylen, Butylen oder eine Alkylen- oder Cycloalkylenbismethylengruppe des Typs $-(-C_pH_{2p}-X-)_t\ C_pH_{2p}-$ sind und
X für Sauerstoff oder Schwefel steht.

Solche Verbindungen sind in EP-0286887 näher beschrieben. Die (Poly-)Di-hydropyridine können im chlorhaltigen Polymer zweckmäßig zu 0,001 bis 5 und insbesondere 0,005 bis 1 Gew.-Teilen, bezogen auf das Polymer, angewandt werden.

Besonders bevorzugt ist Thiodiethylen-bis-[5-methoxycarbonyl-2,6-dimethyl-1,4-dihydro pyridin-3-carboxylat].

**Alkali und Erdalkali-Verbindungen:** Darunter versteht man vornehmlich die Carboxylate der oben beschriebenen Säuren, aber auch entsprechende Oxide bzw. Hydroxide, Carbonate oder basische Carbonate. Es kommen auch deren Gemische mit organischen Säuren in Frage. Beispiele sind NaOH, KOH, CaO, $Ca(OH_2)$, MgO, $Mg(OH)_2$, $CaCO_3$, $MgCO_3$ Dolomit, Huntit, sowie fettsaure Na- K-, Ca. oder Mg-Salze.

Bei Erdalkali- und Zn-Carboxylaten könen auch deren Addukte mit MO oder $M(OH)_2$ (M = Ca, Mg, Sr oder Zn), sogenannte "overbased" Verbindungen, zum Einsatz kommen.

Bevorzugt werden zusätzlich zur erfindungsgemäßen Stabilisatorkombination Alkali-, Erdalkali- und/oder Aluminumcarboxylate eingesetzt, wie z.B. Na, K, Ca oder Aluminiumstearate.

**Perchlorat-Salze:** Beispiele sind diejenigen der Formel $M(ClO_4)_n$ wobei M für Li, Na, K, Mg, Ca, Ba, Zn, Al, Ce oder La steht. Der Index n ist entsprechend der Wertigkeit von M 1, 2 oder 3. Die Perchloratsalze können mit Alkoholen oder Ätheralkoholen komplexiert sein. Das jeweilige Perchlorat kann dabei in verschiedenen gängigen Darreichungsformen eingesetzt werden; z.B als Salz oder wäßrige Lösung aufgezogen auf ein Trägermaterial wie PVC, Ca-Silikat, Zeolithe oder Hydrotalcite, oder durch chemische Reaktion von Hydrotalcit mit Perchlorsäure gewonnen werden.

Die Perchlorate können in einer Menge von beispielsweise 0,001 bis 5, zweckmäßig 0,01 bis 3, besonders bevorzugt 0,01 bis 2 Gew.-Teilen, bezogen auf 100 Gew.-Teile PVC, angewandt werden.

**Hydrotalcite und Zeolithe:** Die chemische Zusammensetzung dieser Verbindungen ist dem Fachmann bekannt, z.B aus den Patentschriften DE 3843581, US 4000100, EP 062813, WO 93/20135.

Verbindungen aus der Reihe der Hydrotalcite können durch die allgemeine Formel III,

$$M^{2+}{}_{1-x} \bullet M^{3+}{}_{x} \bullet (OH)_2 \bullet (A^{n-})_{x/n} \bullet mH_2O \qquad (III)$$

beschrieben werden,
wobei

$M^{2+}$ = eines oder mehrere der Metalle aus der Gruppe Mg, Ca, Sr, Zn oder Sn ist,
$M^{3+}$ = Al oder B ist,
$A^n$ ein Anion mit der Valenz n darstellt,
n eine Zahl von 1-2 ist,

$0 < x \leq 0{,}5$ ist,
m eine Zahl von 0-20 ist.

Bevorzugt ist

$$A^n = OH^-, ClO_4^-, HCO_3^-, CH_3COO^-, C_6H_5COO^-, CO_3^{2-}, SO_4^{--}, HSO_4^- \begin{matrix}COO^-\\ | \\ COO^-\end{matrix}$$

$$, (CHOHCOO)_2^{2-}, (CH_2COO)_2^{2-}, CH_3CHOHCOO^-, HPO_3^{2-} \text{ oder } HPO_4^{2-} \text{ darstellt;}$$

Beispiele für Hydrotalcite sind

$$Al_2O_3.6MgO.CO_2.12H_2O, Mg_{4,5}Al_2(OH)_{13}.CO_3.3,5H_2O, 4MgO.Al_2O_3.CO_2.9H_2O,$$

$$4MgO.Al_2O_3.CO_2.6H_2O, ZnO.3MgO.Al_2O_3.CO_2.8\text{-}9H_2O \text{ und } ZnO.3MgO.Al_2O_3.CO_2.5\text{-}6H_2O.$$

Verbindungen aus der Reihe der Zeolithe (Alkali bzw. Erdalkalialumosilikate) können durch die allgemeine Formel (IV)

$$M_{x/n}[(AlO_2)_x(SiO_2)_y].wH_2O \qquad \textbf{(IV)}$$

beschrieben werden,
worin n die Ladung des Kations M;

M ein Element der ersten oder zweiten Hauptgruppe, wie Li, Na, K, Mg, Ca, Sr oder Ba, oder Zn ist,

y:x eine Zahl von 0,8 bis 15, bevorzugt von 0,8 bis 1,2; und

w eine Zahl von 0 bis 300, bevorzugt von 0,5 bis 30, sind.

Strukturen sind beispielsweise dem "Atlas of Zeolite" von W.M. Meier und D.H. Olson, Butterworth-Heinemann, 3. Aufl. 1992 zu entnehmen.
Beispiele für Zeolithe sind Natriumalumosilikate der Formeln

$$Na_{12}Al_{12}Si_{12}O_{48} \,.\, 27\ H_2O \text{ [Zeolith A]}, Na_6Al_6Si_6O_{24} .\ 2\ NaX \,.\, 7{,}5\ H_2O, X= OH,$$

$$\text{Halogen}, ClO_4 \text{ [Sodalith]}; Na_6Al_6Si_{30}O_{72} \,.\, 24\ H_2O; Na_8Al_8Si_{40}O_{96} .\ 24\ H_2O;$$

$$Na_{16}Al_{16}Si_{24}O_{80} \,.\, 16\ H_2O; Na_{16}Al_{16}Si_{32}O_{96} .\ 16\ H_2O; Na_{56}Al_{56}Si_{136}O_{384} .\ 250\ H_2O$$

$$\text{[Zeolith Y]}, Na_{86}Al_{86}Si_{106}O_{384} \,.\, 264\ H_2O \text{ [Zeolith X]};$$

oder die durch teilweisen bzw. vollständigen Austausch der Na-Atome durch Li-, K-, Mg-, Ca-, Sr- oder Zn-Atome darstellbaren Zeolithe wie

$$(Na,K)_{10}Al_{10}Si_{22}O_{64} .\ 20\ H_2O ; Ca_{4,5}Na_3[(AlO_2)_{12}(SiO_2)_{12}] .\ 30\ H_2O; K_9Na_3[(AlO_2)_{12}(SiO_2)_{12}] .\ 27\ H_2O.$$

Weitere geeignete Zeolithe sind:

$$Na_2O \cdot Al_2O_3(2 \text{ bis } 5)\ SiO_2 \cdot (3{,}5 \text{ bis } 10)\ H_2O \text{ [Zeolith P]}$$

$$Na_2O \cdot Al_2O_3 \cdot 2\ SiO_2 \cdot (3.5\text{-}10) H_2O \text{ (Zeolith MAP)}$$

oder die durch teilweisen bzw. vollständigen Austausch der Na-Atome durch Li-, K- oder H-Atome darstellbaren Zeolithe wie

$(Li,Na,K,H)_{10}Al_{10}Si_{22}O_{64}$. 20 $H_2O$, $K_9Na_3[(AlO_2)_{12}(SiO_2)_{12}]$. 27 $H_2O$, $K_4Al_4Si_4O_{16}\cdot 6H_2O$ [Zeolith K-F], $Na_8Al_8Si_{40}O_{96}$.24 $H_2O$ Zeolith D, wie in Barrer et al.. J. Chem. Soc. **1952**, 1561-71, und in US 2,950,952 beschrieben;

Ferner kommen folgende Zeolithe in Frage:

K-Offretit, wie in EP-A-400,961 beschrieben; Zeolith R, wie in GB 841,812 beschrieben; Zeolith LZ-217, wie in US 4,503,023 beschrieben; Ca-freier Zeolith LZ-218, wie in US 4,333,859 beschrieben; Zeolith T, Zeolith LZ-220, wie in US 4,503,023 beschrieben; $Na_3K_6Al_9Si_{27}O_{72}$.21 $H_2O$ [Zeolith L]; Zeolith LZ-211, wie in US 4,503,023 beschrieben; Zeolith LZ-212, wie in US 4,503,023 beschrieben; Zeolith O, Zeolith LZ-217 , wie in US 4,503,023 beschrieben; Zeolith LZ-219, wie in US 4,503,023 beschrieben; Zeolith Rho, Zeolith LZ-214, wie in US 4,503,023 beschrieben; Zeolith ZK-19, wie in Am. Mineral. **54** 1607 (1969) beschrieben; Zeolith W (K-M), wie in Barrer et al. J. Chem. Soc.- **1956**, 2882, beschrieben; $Na_{30}Al_{30}Si_{66}O_{192}$. 98 $H_2O$ [Zeolith ZK-5, Zeolith Q]

Besonders bevorzugt werden Zeolith P-Typen der Formel IV verwandt, worin x 2 bis 5 und y 3.5 bis 10 sind, ganz besonders bevorzugt Zeolith MAP der Formel IV, worin x 2 und y 3.5 bis 10 sind. Insbesondere handelt es sich um Zeolith Na-P, d.h. M steht für Na. Dieser Zeolith tritt im allgemeinen in den Varianten Na-P-1, NaP-2 und Na-P-3 auf, die sich durch ihre kubische, tetragonale oder orthorhombische Struktur unterscheiden (R.M.Barrer, B.M.Munday, J.Chem.Soc. A**1971**, 2909-14). In der eben genannten Literatur ist auch die Herstellung von Zeolith P-1 und P-2 beschrieben. Zeolith P-3 ist danach sehr selten und daher kaum von praktischem Interesse. Die Struktur des Zeolith P-1 entspricht der aus dem obengenannten Atlas of Zeolite Structures bekannten Gismonditstruktur. In neuerer Literatur (EP-A-384 070) wird zwischen kubischem (Zeolith B oder $P_C$) und tetragonalem (Zeolith $P_1$) Zeolith vom P-Typ unterschieden. Dort werden auch neuere Zeolithe des P-Typs mit Si:Al Verhältnissen unter 1,07:1 genannt. Hierbei handelt es sich um Zeolithe mit der Bezeichnung MAP oder MA-P für "Maximum Aluminium P". Je nach Herstellungsverfahren kann Zeolith P geringe Anteile anderer Zeolithe enthalten. Sehr reiner Zeolith P ist in WO 94/26662 beschrieben worden.

Im Rahmen der Erfindung lassen sich auch solche feinteiligen, wasserunlöslichen Natriumalumosilikate verwenden, die in Gegenwart von wasserlöslichen anorganischen oder organischen Dispergiermitteln gefällt und kristallisiert wurden. Diese können in beliebiger Weise vor oder während der Fällung bzw. Kristallisation in das Reaktionsgemisch eingebracht werden.

Bevorzugt sind Na-Zeolith A und Na-Zeolith P.

Die Hydrotalcite und Zeolithe können natürlich vorkommende Mineralien oder synthetisch hergestellte Verbindungen sein.

Die Hydrotalcite und/oder Zeolithe können in Mengen von beispielsweise 0,1 bis 50, zweckmäßig 0,1 bis 10 und insbesondere 0,1 bis 5 Gew.-Teilen, bezogen auf 100 Gew.-Teile halogenhaltiges Polymer angewandt werden.

**Alkalialumocarbonate (Dawsonite):** Diese Verbindungen lassen sich durch die Formel

$$\{(M_2O)_m \cdot (Al_2O_3)_n \cdot Z_o \cdot pH_2O\} \qquad \text{(V)},$$

darstellen

worin M H, Li, Na, K, $Mg_{1/2}$, $Ca_{1/2}$, $Sr_{1/2}$ oder $Zn_{1/2}$; Z $CO_2$, $SO_2$, $(Cl_2O_7)_{1/2}$, $B_4O_6$, $S_2O_2$ (Thiosulfat) oder $C_2O_2$ (Oxalat); m, wenn M $Mg_{1/2}$ oder $Ca_{1/2}$ ist, eine Zahl zwischen 1 und 2, in allen anderen Fällen eine Zahl zwischen 1 und 3; n eine Zahl zwischen 1 und 4; o eine Zahl zwischen 2 und 4; und p eine Zahl zwischen 0 und 30 ist.

Die verwendbaren Alumosalz-Verbindungen der Formel (V) können natürlich vorkommende Mineralien sein oder synthetisch hergestellte Verbindungen. Die Metalle können partiell gegeneinander ausgetauscht sein. Die genannten Alumosalz-Verbindungen sind kristallin, teilkristallin oder amorph oder können als getrocknetes Gel vorliegen. Die Alumosalzverbindungen können auch in selteneren, kristallinen Modifikationen vorliegen. Ein Verfahren zur Herstellung solcher Verbindungen ist in EP 394670 angegeben. Beispiele für natürlich vorkommende Alumosalz-Verbindungen sind Indigirit, Tunisit, Alumohydrocalcit, Para-Alumohydrocalcit, Strontiodresserit und Hydro-Strontiodresserit. Weitere Beispiele für Alumosalz-Verbindungen sind Kaliumalumocarbonat

$\{(K_2O).(Al_2O_3).(CO_2)_2.2H_2O\}$, Natriumalumothiosulfat $\{(Na_2O).(Al_2O_3).(S_2O_2)_2.2H_2O\}$, Kaliumalumosulfit $\{(K_2O).(Al_2O_3).(SO_2)_2.2H_2O\}$, Calciumalumooxalat $\{(CaO).(Al_2O_3).(C_2O_2)_2.5H_2O\}$, Magnesiumalumotetraborat $\{(MgO).(Al_2O_3).(B_4O_6)_2.5H_2O)$, $\{([Mg_{0,2}Na_{0,6}]_2O).(Al_2O_3).(CO_2)_2.4,1H_2O\}$, $\{([Mg_{0,2}Na_{0,6}]_2O).(Al_2O_3).(CO_2)_2.4,3H_2O\}$ und $\{([Mg_{0,3}Na_{0,4}]_2O).(Al_2O_3).(CO_2)_{2,2}.4,9H_2O\}$.

Die gemischten Alumosalz-Verbindungen können nach an sich bekannten Verfahren durch Kationenaustausch, bevorzugt aus den Alkali-Alumosalz-Verbindungen oder durch Kombinationsfällung (siehe beispielsweise US 5,055,284) erhalten werden.

Bevorzugt sind Alumosalz-Verbindungen der obigen Formel, worin M Na oder K; Z $CO_2$, $SO_2$ oder $(Cl_2O_7)_{1/2}$; m 1-3; n 1-4; o 2-4 und p 0-20 bedeuten. Besonders bevorzugt bedeutet Z $CO_2$.

Weiter sind Verbindungen bevorzugt, welche sich durch folgende Formeln darstellen lassen:

$$M_2O.Al_2O_3.(CO_2)_2.\ pH_2O \quad \text{(Ia)},$$

$$(M_2O)_2.(Al_2O_3)_2.(CO_2)_2.\ pH_2O \quad \text{(Ib)},$$

$$M_2O.(Al_2O_3)_2.(CO_2)_2.\ pH_2O \quad \text{(Ic)}$$

wobei M ein Metall wie Na, K, $Mg_{1/2}$, $Ca_{1/2}$, $Sr_{1/2}$ oder $Zn_{1/2}$ und p eine Zahl von 0 bis 12 bedeutet.

Besonders bevorzugt ist Natriumalumodihydroxycarbonat (DASC) und die homologe Kaliumverbindung (DAPC).

Die Dawsonite können in einer Menge von beispielsweise 0,01 bis 50, zweckmäßig 0,1 bis 10, besonders bevorzugt 0,1 bis 5 Gew.-Teilen, bezogen auf 100 Gew.-Teile halogenhaltiges Polymer angewandt werden.

Die erfindungsgemäße Stabilisatorkombination kann zusammen mit weiteren Zusätzen verwandt werden, die für die Verarbeitung und Stabilisierung chlorhaltiger Polymere üblich sind, wie z.B.:

**Antioxidantien:** Als solche kommen beispielsweise in Betracht:

1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.

4. Tocopherole, z.B. α-Tocopherol, β-Tocopherol, γ-Tocopherol, δ-Tocopherol und Mischungen davon (Vitamin E).

5. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.

6. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methyl-phenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercapto-butan, 1,1 , 5,5-Tetra-(5-tert-butyl-4-hydroxy-2-methylphenyl)-pentan.

7. O-,N- und S-Benzylverbindungen, z.B. 3,5,3',5'-Tetra-tert-butyl-4,4'-dihydroxy-dibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-di-tert-butyl-4-hydroxybenzyl-mercaptoacetat.

8. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2'2-bis-(3'5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.

9. Hydroxybenzyl-Aromaten, z. B. 1 ,3,5-Tris-(3 ,5-di-tert-butyl-4-hydroxybenzyl)-2,4,6-trimethylbenzol, 1 , 4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

10. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxy-anilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanu rat, 1 ,3,5-Tris-(4-tert-butyl-3-hydroxy-2,6-dimethylbenzyl) isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.

11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

13. Ester der β-(3,5-Di-tert-butyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

14. Ester der β-(5-tert-Butyl-4-hydroxy-3-methylphenyl)-propionsäure mit ein-oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy) ethyl-isocyanurat, N, N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

15. Ester der β-13,5-Dicyclohexyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy) ethyl-isocyanurat, N, N'-Bis-(hydroxyethyl)-oxalsäu rediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

17. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

Gegebenenfalls kann auch ein Gemisch von Antioxidantien unterschiedlicher Struktur eingesetzt werden.

Die Antioxidantien können in einer Menge von beispielsweise 0,01 bis 10, zweckmäßig 0,05 bis 10 und insbesondere 0,05 bis 5 Gew.-Teilen, bezogen auf 100 Gew.-Teile PVC, angewandt werden.

**UV-Absorber und Lichtschutzmittel:** Beispiele dafür sind:

1.2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1 , 3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis-($\alpha$,$\alpha$-dimethylbenzyl)-2'-hydroxyphenyl)-benzotriazol, Mischung aus 2-(3'-tert-Butyl-2'-hydroxy-5'-(2-octyloxycarbonylethyl)phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy) carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300;

$$\left[RCH_2CH_2COO(CH_2)_3\right]_2\!\!-$$

mit R = 3'-tert-Butyl-4'-hydroxy-5'-2H-benzotriazol-2-yl-phenyl.

2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

3. Ester von gegebenenfalls substituierten Benzoesäuren, wie z.B. 4-tert-Butyl-phenyl-salicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

4. Acrylate, wie z.B. $\alpha$-Cyan-$\beta$,$\beta$-diphenylacrylsäure-ethylester bzw. - isooctylester, $\alpha$-Carbomethoxy-zimtsäuremethylester, $\alpha$-Cyano-$\beta$-methyl-p-methoxy-zimtsäuremethylester bzw. -butylester, $\alpha$-Carbomethoxy-p-methoxy-zimtsäure-methylester, N-($\beta$-Carbomethoxy-$\beta$-cyanovinyl)-2-methyl-indolin.

5. Nickelverbindungen, wie z.B. Nickelkomplexe des 2,2'-Thio-bis-[4-(1,1,3,3-tetra-methylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzyl phosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

6. Sterisch gehinderte Amine, wie z.B. Bis-(2,2,6,6-tetramethyl-piperidyl)-sebacat, Bis-(2,2,6,6-tetramethylpiperidyl)-succinat, Bis-(1,2,2,6,6-pentamethylpiperidyl)-sebacat, n-Butyl-3,5-di-tert-butyl-4-hydroxybenzylmalonsäure-bis(1,2,2,6,6-pentamethylpiperidyl)ester, Kondensationsprodukt aus 1-Hydroxyethyl-2,2,6,6-tetramethyl-4-hydroxy-piperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin' Tris-(2,2'6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetrarpethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion,3-Dodecyl-1-(1 ,2,2,6,6-pentamethyl-4-piperidyl)-pyrrolidin-2,5-dion.

7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propoxyphenyl)-6-(2,4-dimethylphenyl)-1,3,5-triazin, 2-(2-Hydroxy-4-octyloxyphenyl)-4,6-bis(4-methylphenyl)-,3,5-triazin, 2-(2-Hydroxy-4-dodecyloxyphenyl)-4,6-bis(2,4-dimethylphenyl)-1,3,5-triazin, 2-[2-Hydroxy-4-(2-hydroxy-3-butyloxy-propyloxy)phenyl]-4,6-bis(2 , 4-dimethylphenyl)-1,3,5-triazin, 2-[2-Hydroxy-4-(2-hydroxy-3-octyloxy-propoxy)phenyl]-4,6-bis(2,4-di-methylphenyl)-1,3,5-triazin.

Bevorzugt wird die erfindungsgemäße Stabilisatormischung mit Alkali- oder Erdalkalicarboxylaten, besonders Calciumcarboxylaten, mit Aluminiumcarboxylaten, mit 1,3-Dicarbonylverbindungen, mit Dihydropyridinen, mit Phosphiten oder Kombinationen dieser Stoffe.

**Weichmacher:** Als organische Weichmacher kommen beispielsweise solche aus den folgenden Gruppen in Betracht:

A) Phthalsäureester: Beispiele für solche Weichmacher sind Dimethyl-, Diethyl-, Dibutyl-, Dihexyl-, Di-2-ethylhexyl-, Di-n-octyl-, Di-iso-octyl-, Di-iso-nonyl-, Di-iso-decyl-, Di-iso-tridecyl-, Dicyclohexyl-, Di-methylcyclohexyl-, Dimethylglycol-, Dibutylglycol-, Benzylbutyl- und Diphenyl-phthalat sowie Mischungen von Phthalaten wie $C_{7-9}$- und $C_{9-11}$-Alkylphthalate aus überwiegend linearen Alkoholen, $C_{6-10}$-n-Alkylphthalate und $C_{8-10}$-n-Alkylphthalate. Bevorzugt sind davon Dibutyl-, Dihexyl-, Di-2-ethylhexyl-, Di-n-octyl-, Di-iso-octyl-, Di-iso-nonyl-, Di-iso-decyl-, Di-iso-tridecyl-und Benzylbutyl-phthalat sowie die genannten Mischungen von Alkylphthalaten. Besonders bevorzugt sind Di-2-ethylhexyl-, Di-iso-nonyl- und Di-iso-decylphthalat, die auch unter den gebräuchlichen Abkürzungen DOP (Dioctylphthalat, Di-2--ethylhexyl-phthalat), DINP (Diisononylphthalat), DIDP (Diisodecylphthalat) bekannt sind.

B) Ester aliphatischer Dicarbonsäuren, insbesondere Ester von Adipin-, Azelain-und Sebazinsäure
Beispiele für solche Weichmacher sind Di-2-ethylhexyladipat, Di-isooctyladipat (Gemisch), Di-iso-nonyladipat (Gemisch), Di-iso-decyladipat (Gemisch), Benzylbutyladipat, Benzyloctyladipat, Di-2-ethylhexylazelat, Di-2-ethylhexylsebacat und Di-iso-decylsebacat (Gemisch). Bevorzugt sind Di-2-ethylhexyladipat und Di-isooctyladipat.

C) Trimellithsäureester, beispielsweise Tri-2-ethylhexyltrimellithat, Tri-iso-decyltrimellithat (Gemisch), Tri-iso-tridecyltrimellithat, Tri-iso-octyltrimellithat (Gemisch) sowie Tri-$C_{6-8}$-alkyl, Tri-$C_{6-10}$-alkyl-, Tri-$C_{7-9}$-alkyl- und Tri-$C_{9-11}$-alkyl-trimellithate. Die letztgenannten Trimellithate entstehen durch Veresterung der Trimellithsäure mit den entsprechenden Alkanolgemischen. Bevorzugte Trimellithate sind Tri-2-ethylhexyltrimellithat und die genannten Trimellithate aus Alkanolgemischen. Gebräuchliche Abkürzungen sind TOTM (Trioctyltrimellitat, Tri--2-ethylhexyl-trimellitat), TIDTM (Triisodecyltrimellitat) und TITDTM (Triisotridecyl-trimellitat).

D) Epoxyweichmacher: In der Hauptsache sind das epoxidierte ungesättigte Fettsäuren wie z.B. epoxidiertes Sojabohnenöl.

E) Polymerweichmacher: Die gebräuchlichsten Ausgangsmaterialien für die Herstellung der Polyesterweichmacher sind: Dicarbonsäuren wie Adipin-, Phthal-, Azelain- und Sebacinsäure; Diole wie 1,2-Propandiol, 1,3-Butandiol, 1,4-Butandiol, 1,6-Hexandiol, Neopentylglycol und Diethylenglykol.

F) Phosphorsäureester: Beispiele für solche Phosphorsäureester sind Tributylphosphat, Tri-2-ethylbutylphosphat, Tri-2-ethylhexylphosphat, Trichlorethylphosphat, 2-Ethyl-hexyl-di-phenylphosphat, Kresyldiphenylphosphat, Triphenylphosphat, Trikresylphosphat und Trixylenylphosphat. Bevorzugt sind Tri-2-ethylhexyl-phosphat sowie ®Reofos 50 und 95 (ex. Fa. FMC).

G) Chlorierte Kohlenwasserstoffe (Paraffine)

H) Kohlenwasserstoffe

I) Monoester, z.B. Butyloleat, Phenoxyethyloleat, Tetrahydrofurfuryloleat und Alkylsulfonsäureester.

J) Glykolester, z.B. Diglykolbenzoate.

Definitionen und Beispiele für Weichmacher der Gruppen A) bis J) sind den folgenden Handbüchern zu entnehmen:
"Taschenbuch der Kunststoffadditive", Herausgeber R. Gächter und H. Müller, Carl Hanser Verlag, 1989, Kapitel 5 S 341-442.
"PVC Technology", Herausgeber W.V. Titow, 4th. Ed., Elsevier Publishers, 1984, Kapitel 6. Seiten 147-180.
Es können auch Mischungen unterschiedlicher Weichmacher verwandt werden.

Die Weichmacher können in einer Menge von beispielsweise 5 bis 120, zweckmäßig 10 bis 100, Gew.-Teilen, bezogen auf 100 Gew.-Teile PVC, angewandt werden.

Als **Gleitmittel** kommen beispielsweise in Betracht: Montanwachs, Fettsäureester, PE-Wachse, Amidwachse, Chlorparaffine, Glycerinester oder Erdalkaliseifen bzw. Gleitmittel auf Silikonbasis wie in EP 225261 beschrieben. Verwendbare Gleitmittel sind auch in "Taschenbuch der Kunststoffadditive", Herausgeber R. Gächter und H. Müller, Carl Hanser Verlag, 3.Auflage, 1989, Seiten 478-488 beschrieben.

**Füllstoffe:** Füllstoffe ("Handbook of PVC-Formulating" von E. J. Wickson, John Wiley & Sons, New York 1993S. 393-449) und Verstärkungsmittel ("Taschenbuch der Kunststoffadditive", Herausgeber R. Gächter und H. Müller, Carl Hanser Verlag, 3.Auflage, 1989, Seiten 549-615) können z.B. sein: Calciumcarbonat, Dolomit, Wollastonit, Magnesiumoxid, Magnesiumhydroxid, Silikate, Glasfasern, Talk, Kaolin, Kreide, Glimmer, Metalloxide und -hydroxide, Ruß oder Graphit). Bevorzugt ist Kreide.

**Pigmente:** Geeignete Stoffe sind dem Fachmann bekannt. Beispiele für anorganische Pigmente sind $TiO_2$, Ruß, $Fe_2O_3$, $Sb_2O_3$, $(Ti,Ba,Sb)O_2$, $Cr_2O_3$, Spinelle wie Cobaltblau und Cobaltgrün, Cd(S,Se), Ultramarinblau. Bevorzugt ist $TiO_2$, auch in mikronisierter Form. Organische Pigmente sind z.B. Azopigmente, Phthalocyaninpigmente, Chinacridonpigmente, Perylenpigmente, Pyrrolpyrilidonpigmente und Anthrachinonpigmente. Weitere Einzelheiten sind in "Handbook of PVC Formulating", E.J. Wickson, John Wiley & Sons, New York 1993, S. 449 - 474, zu finden.

Beispiele für die zu stabilisierenden **chlorhaltigen Polymere** oder deren Recyclate sind: Polymere des Vinylchlorides, Vinylharze, enthaltend Vinylchlorideinheiten in deren Struktur, wie Copolymere des Vinylchlorids und Vinylester von aliphatischen Säuren, insbesondere Vinylacetat, Copolymere des Vinylchlorids mit Estern der Acryl- und Methycrylsäure und mit Acrylnitril, Copolymere des Vinylchlorids mit Dienverbindungen und ungesättigten Dicarbonsäuren oder deren Anhydride, wie Copolymere des Vinylchlorids mit Diethylmaleat, Diethylfumarat oder Maleinsäureanhydrid, nachchlorierte Polymere und Copolymere des Vinylchlorids, Copolymere des Vinylchlorids und Vinylidenchlorids mit ungesättigten Aldehyden, Ketonen und anderen, wie Acrolein, Crotonaldehyd, Vinylmethylketon, Vinylmethylether, Vinylisobutylether und ähnliche; Polymere des Vinylidenchlorids und Copolymere desselben mit Vinylchlorid und anderen polymerisierbaren Verbindungen; Polymere des Vinylchloracetates und Dichlordivinylethers; chlorierte Polymere des Vinylacetates, chlorierte polymerische Ester der Acrylsäure und der alpha-substituierten Acrylsäure; Polymere von chlorierten Styrolen, zum Beispiel Dichlorstyrol; chlorierte Gummis; chlorierte Polymere des Ethylens; Polymere und nachchlorierte Polymere von Chlorbutadiens und deren Copolymere mit Vinylchlorid, chlorierte Natur- und Synthesekautschuke, sowie Mischungen der genannten Polymere unter sich oder mit anderen polymerisierbaren Verbindungen.

Im Rahmen dieser Erfindung sind unter PVC auch Copolymerisate mit polymerisierbaren Verbindungen wie Acrylnitril, Vinylacetat oder ABS zu verstehen, wobei es sich um Suspensions-, Masse- oder Emulsionspolymerisate handeln kann. Bevorzugt ist PVC Homopolymer auch in Kombination mit Polyacrylaten.

Ferner sind umfaßt die Pfropfpolymerisate von PVC mit EVA, ABS und MBS. Bevorzugte Substrate sind auch Mischungen der vorstehend genannten Homo-und Copolymerisate, insbesondere Vinylchlorid-Homopolymerisate, mit anderen thermoplastischen oder/und elastomeren Polymeren, insbesondere Blends mit ABS, MBS, NBR, SAN, EVA, CPE, MBAS, PMA, PMMA, EPDM und Polylactonen.

Bevorzugt sind Suspensions- und Massepolymere, sowie Emulsionspolymere.

Als chlorhaltiges Polymerisat ist Polyvinylchlorid besonders bevorzugt, insbesondere als Suspensionspolymerisat und Massepolymerisat.

Weiterhin kommen zur Stabilisierung im Rahmen dieser Erfindung auch insbesondere Recyclate chlorhaltiger Polymere in Frage, wobei es sich hierbei um die oben näher beschriebenen Polymere handelt, welche durch Verarbeitung, Gebrauch oder Lagerung eine Schädigung erfahren haben. Besonders bevorzugt ist PVC-Recyclat. In den Recyclaten können auch kleine Mengen an Fremdstoffen enthalten sein, wie z.B. Papier, Pigmente, Klebstoffe, die oft schwierig zu entfernen sind. Diese Fremdstoffe können auch aus dem Kontakt mit diversen Stoffen während des Gebrauchs oder der Aufarbeitung stammen, wie z.B. Treibstoffreste, Lackanteile, Metallspuren und Initiatorreste.

Die Anwendung der Stabilisatormischung geschieht auf dem Fachmann bekannte Weise.

Die Stabilisatormischung kann z.B. auch zusammen mit konventionellen Zusätzen vor der eigentlichen Verwendung formgebend zu beispielsweise Granulat oder Extrudat oder einer Paste verarbeitet werden.

Ein weiterer Gegenstand der vorliegenden Erfindung ist die Verwendung der soeben beschriebenen Stabilisatormischung - auch als Granulat, Extrudat oder Paste auch in Verbindung mit Gleitmitteln (sog. *one-pack)* - zur Stabilisierung eines halogenhaltigen Polymers oder Polymer-Recyclates. Für die einzelnen Stabilisatoren sowie das halogenhaltige Polymer selbst gelten die vorstehend erläuterten Bevorzugungen, ebenso kann zusätzlich einer der oben beschriebenen weiteren Bestandteile verwandt werden.

Die Stabilisatormischung kann dem Polymeren auf bekannte Weise zugegeben werden, wobei man unter Verwendung bekannter Vorrichtungen wie Mischern, Knetern, Extrudern, Mühlen und dergleichen, die genannten Stabilisatoren und gegebenenfalls weitere Zusätze mit dem halogenhaltigen Polymer vermischt. Hierbei können die Stabilisatoren einzeln oder in Mischung zugegeben werden oder auch in Form sogenannter Masterbatches. Die Erfindung betrifft somit auch ein Verfahren zur Stabilisierung von halogenhaltigem Polymer, dadurch gekennzeichnet, daß man

unter Verwendung von Vorrichtungen, wie Kalandern, Mischern, Knetern, Extrudern und dergleichen, die Stabilisatorkomponenten und gegebenenfalls weitere Zusätze mit dem PVC vermischt.

Die Erfindung betrifft auch die solchermaßen stabilisierten Polymerzusammensetzungen enthaltend die erfindungsgemäße Stabilisatormischung. Sie können auf bekannte Weisen in die gewünschte Form gebracht werden. Solche Verfahren sind beispielsweise Kalandrieren, Extrudieren, Spritzgießen, Sintern oder Spinnen, ferner Extrusions-Blasen oder eine Verarbeitung nach dem Plastisol-Verfahren. Die Polymerzusammensetzungen können auch zu Schaumstoffen verarbeitet werden.

Die Erfindung betrifft auch die Verwendung der stabilisierten Polymerzusammenetzungen zur Herstellung von aus halogenhaltigem Polymer herstellbaren Formkörpern. Die erfindungsgemäßen Polymerzusammensetzungen eignen sich für Halbhart- und Weich-Rezepturen, wie z.B. für Weichrezepturen für Drahtummantelungen und Kabelisolierungen. In Form von Halbhart-Rezepturen eignen sich die erfindungsgemäßen Polymerzusammensetzungen für Dekorations-folien, Schaumstoffe, Agrarfolien, Schläuche, Dichtungsprofile, Bürofolien, extrudierte Profile und Platten, Fußbodenfolien und Platten, Streichwaren und Kunstleder sowie Crashpad Folien (für die Verwendung im Automobilbereich).

In Form von Hart-Rezepturen eignen sich die erfindungsgemäßen Polymerzusammensetzungen für Hohlkörper (Flaschen), Verpackungsfolien (Tiefziehfolien), Blasfolien, Crash pad-Folien (Automobile), Rohre, Schaumstoffe, Schwerprofile (Fensterrahmen), Lichtwandprofile, Bauprofile, Sidings, Fittings, und Apparatur-Gehäuse (Computer, Haushaltsgeräte) sowie andere Spritzgußartikel.

Beispiele für die Anwendung der erfindungsgemäß stabilisierten Polymerzusammensetzungen als Plastisol sind Kunstleder, Fußböden, Textilbeschichtungen, Tapeten, Coil-Coatings und Unterbodenschutz für Kraftfahrzeuge.

Beispiele für Sinter-Anwendungen der erfindungsgemäß stabilisierten Polymerzusammensetzungen sind Slush, Slush Mould und Coil-Coatings.

Bevorzugt sind PVC-Hartschaumstoffe und PVC-Rohre für Trink- oder Abwasser, Druckrohre, Gasrohre, Kabelkanal- und Kabelschutzrohre, Rohre für Industrieleitungen, Sickerrohre, Abflußrohre, Dachrinnenrohre und Drainagerohre. Näheres hierzu vgl. "Kunststoffhandbuch PVC" Bd. 2/2, W. Becker, H. Braun, 2. Aufl 1985, Carl Hanser Verlag, S. 1235-1277.

Die **Herstellung** der erfindungsgemäßen Amine erfolgt nach allgemein bekannten Methoden. Die Triazinylderivate zum Beispiel werden durch Umsetzung von Cyanurchlorid mit Dialkylaminoethylaminen in Gegenwart von Natronlauge erhalten. Dialkylaminoalkyl-carbonsäureesteramide werden zum Teil durch Umsetzung der Carbonsäuren mit den entsprechenden Aminoalkoholen bzw, durch Aminolyse von Carbonsäureestern mit den jeweiligen Dialkylamino-alkylaminenhergestellt.

Die folgenden **Beispiele** erläutern die Erfindung weiter, ohne sie jedoch zu beschränken. Teile- und Prozentangaben beziehen sich - wie auch in der übrigen Beschreibung - sofern nicht anders angegeben, auf das Gewicht.

***Beispiel 1: Tris.[N,N.di.i.propyl-ethylen-diamino]-triazin***

44.3 g (0.24 mol) Cyanurchlorid werden in 300 ml Wasser supendiert und unter Rühren im Verlauf von 30 Min. mit 103.9 g (0.72 mol) N,N-di-i-propyl-ethylendiamin versetzt. Die erhaltene verdickte Suspension wird unter Rühren bei Raumterperatur im Verlauf von 4 Stunden mit einer Lösung von 19.2 g NaOH in 80 ml Wasser versetzt. Nach Zugabe von 200 ml Toluol werden unter kräftigem Rühren bei 85-86°C im Verlauf von 1.5 Stunden weitere 9.2 g NaOH in 40 ml Wasser zugegeben (pH-Wert der Mischung: 10.3) . Nach Abkühlen und Abtrennen der Wasser-Phase wird die Toluol-Phase nachgewaschen, über $Na_2SO_4$ (wasserfrei) getrocknet, filtriert und das Lösungsmittel Im Vakuum bei 80°C abdestilliert.

Ausbeute: 105.6 g (87% d.Th.) hellbraune, hochviskose Flüssigkeit. Die HCl Titration zeigt eine Reinheit von 99%.

***Beispiel 2: Bis-[N,N-di-i-propyl-aminoethyl]-oxamid***

Zu einer Mischung aus 50.4 g (0.4 mol) Oxalsäure Dihydrat und 0.41 g p-Toluolsulfonsäure (PTS) in 500 ml Toluol werden unter Rühren im Verlauf von 20 Minuten 115.4 g (0.8 mol) N,N-Di-i-propyl-ethylendiamin zugetropft, wobei die Temperatur auf 40°C steigt. Das Toluol/Wasser-Gemisch wird abdestilliert, der Ansatz mit 200 ml Xylol und 0.4 g PTS versetzt und am Wasserabscheider zum Abtrennen des Reaktionswassers zum Sieden erhitzt. Die organische Phase wird mit gesättigter Natriumhydrogencarbonatlösung ausgeschüttelt, mit wasserfreiem Natriumsulfat getrocknet und zur Trockne eingedampft. Der erhaltene Rückstand wird aus Petrolether umkristalllisiert.

Ausbeute: 57.2 g (42 % d.Th) farblose Kristalle. Fp. 73-74°C.

In der nachstehenden Tabelle 1 sind weitere erfindungsgemäße Amine zusammengestellt, die nach den vorstehend beschriebenen Methoden bzw. durch Aminolyse entsprechender Ester hergestellt werden.

| | Tabelle 1 | | |
|---|---|---|---|
| Beispiel | Struktur | Fp [°C] bzw. Kp [°C] | $n^{D}_{20}$ bzw. Zustand |
| 3 | 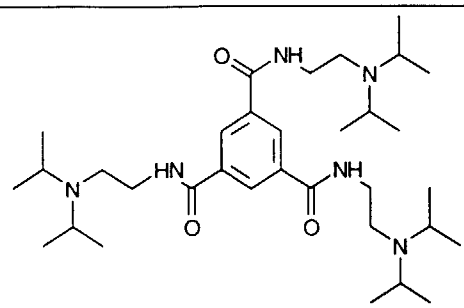 | 265-73 | fest |
| 4 | 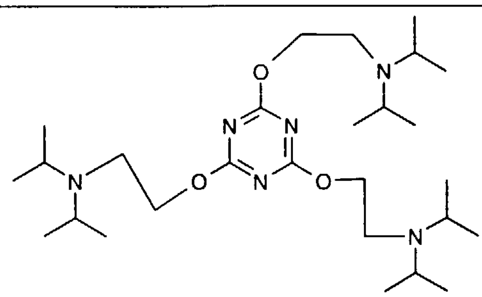 | - | viskoses Harz |
| 5 | 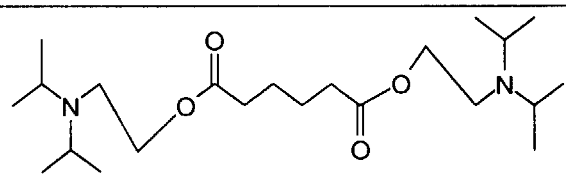 | - | 1.4561 flüssig |
| 6 | 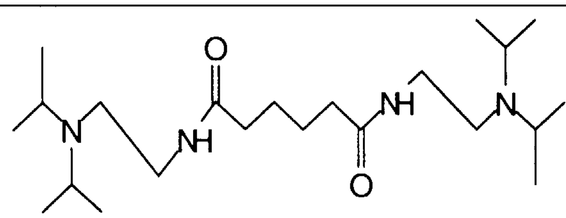 | 110-12 | fest |
| 7 | 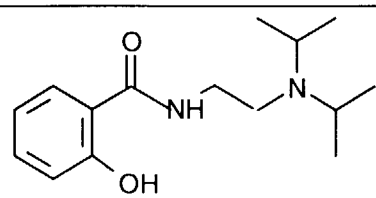 | - | viskoses Öl |
| 8 | 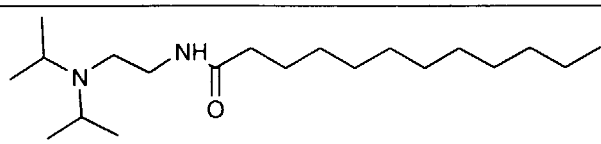 | 160-62/ 0.1 bar | 1.4612 flüssig |

***Beispiel 9: Statischer Hitzetest***

Zur Herstellung der Stabilisatoren I (ohne), und II (mit Coadditiv) wird ein Zink/Calcium-Stearatgemisch mit einem handelsüblichen

1.3-Diketon (Stab I) und weiter mit der Aminverbindung 5 (Tabelle 1) im Verhältnis 8/2/1,5 (Stab II) auf einem

Taumelmischer über eine Zeit von 1,5 Stunden intensiv gemischt.

Von diesen Stabilisatormischungen werden jeweils 1.0 Teile (Stab 1) und 1,15 Teile (Stab II) mit 100 Teilen S-PVC (K-Wert 70) und 21 Teilen einer Mischung aus Dioctylphthalat und epoxidiertem Sojaöl und einem handelsüblichen flüssigen Aryl-Alkyl-Phosphit vermischt und 5 Minuten bei 190°C auf einem Mischwalzwerk plastifiziert. Aus den so erhaltenen Folien (Stärke 0.2 mm) werden Prüfstreifen geschnitten und in einem Mathis-Thermotakter bei 180°C über den in den nachstehenden Tabellen angegebenen Zeitraum thermisch belastet. Anschließend wird der Yellowness Index (YI) nach ASTM-1925-70 gemessen.

Je niedriger der gefundene YI-Wert ist, desto wirksamer verhindert das Stabilisatorsystem die Vergilbung und damit die Schädigung des Materials. Die Langzeitthermostabilität des stabilisierten Polymeren ist auch aus dem plötzlichen Einsetzen einer massiven Verfärbung zu erkennen.

Ein Stabilisator ist desto wirksamer, je länger diese Verfärbung unter thermischer Belastung verzögert wird, oder je niedriger die Anfangsverfärbung und je besser die Farbhaltung (geringe Mittelverfärbung) ist.

Tabelle 2:

| Statischer Hitzetest bei 180°C | | | | | | | |
|---|---|---|---|---|---|---|---|
| YI der Prüfkörper und Abbruchzeitwerte | | | | | | | |
| Stabilisator | Testdauer [Minuten] | | | | | | Abbruchzeit [Minuten] |
| | 0 | 6 | 12 | 18 | 24 | 30 | |
| I | 3.8 | 4.1 | 5.0 | 29.8 | -schwarz- | | 16 |
| II | 3.8 | 4.6 | 4.8 | 5.5 | 9.8 | 25.0 | 32 |

Zur Herstellung der Stabilisatormischungen III und IV wird ein Zink/Aluminum-Stearat-Gemisch mit einem handelsüblichen 1.3-Diketon (Stab III) und weiter mit der Amin-Verbindung gemäß Beispiel 1 im Verhältnis 8/2/1,5 auf einem Taumelmischer im Verlauf von 1,5 Stunden intensiv gemischt.

Von diesen Stabilisatormischungen werden jeweils 1,0 (Stab III) bzw. 1,15 Teile (Stab IV) mit 100 Teilen S-PVC (K-Wert 70) und 21 Teilen einer Mischung aus Dioctylphthalat, epoxidiertem Sojabohnenenöl und einem handelsüblichen flüssigen Aryl-Alkyl-Phosphit vermischt und 5 Minuten bei 180°C auf einem Mischwalzwerk plastifiziert. Aus den so erhaltenen Folien werden Prüfmuster geschnitten und in einem Mathis-Thermotakter bei 180°C thermisch belastet.

Anschließend wird der Yellowness Index nach ASTM 1925-70 gemessen.

Tabelle 3:

| Statischer Hitzetest bei 180°C | | | | | | | |
|---|---|---|---|---|---|---|---|
| YI der Prüfkörper und Abbruchzeitwerte | | | | | | | |
| Stabilisator | Testdauer [Minuten] | | | | | | Abbruchzeit [Minuten] |
| | 0 | 6 | 12 | 18 | 24 | 30 | |
| III | 4.5 | 5.0 | 5.3 | -----schwarz----- | | | 13 |
| IV | 5.5 | 6.0 | 6.3 | 6.6 | 7.4 | 8.3 | 77 |

**Patentansprüche**

1. Verbindungen der Formel I und II

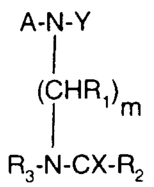

(I) ,

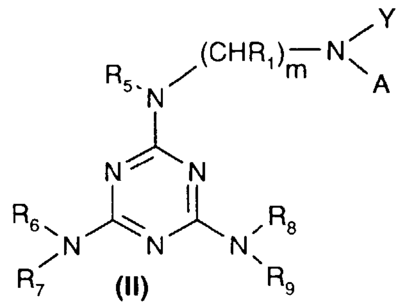

(II)

worin A und Y unabhängig voneinander $C_1$-$C_8$ -Alkyl, $C_3$-$C_8$-Alkenyl, $C_5$-$C_8$-Cycloalkyl oder $C_7$-$C_9$-Phenylalkyl sind oder zusammen gegebenenfalls durch O, NH oder N-$CH_3$ unterbrochenes $C_2$-$C_5$-Alkylen bilden,

$R_1$: H oder $C_1$-$C_4$-Alkyl,

$R_2$ : $C_1$-$C_8$-Alkyl, $NH_2$ ,

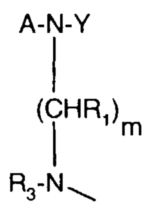

, $R_4$-O,

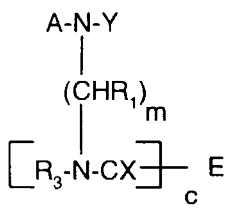

,

Phenyl oder mit OH, $C_1$-$C_4$-Alkyl-O, $C_1$-$C_4$-Alkyl substituiertes Phenyl,

$R_3$: H, $C_1$-$C_8$-Alkyl, $C_3$-$C_8$-Alkenyl, $C_5$-$C_8$-Cycloalkyl,

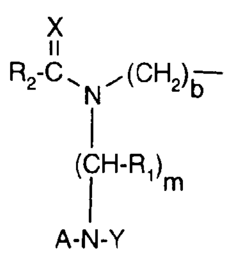

,

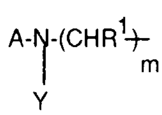

,

$R_4$ : $C_1$-$C_4$-Alkyl, Cyclohexyl, Benzyl;

E : -$(CH_2)_a$-, -CH=CH-,

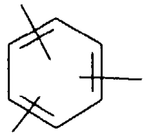 , 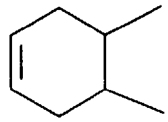 , 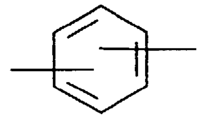 , 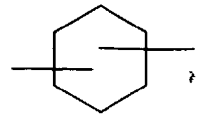 ;

-CH(OH)-CH(OH)-, -NH $(CH_2)_b$NH-;

X: O oder S;

a: 0 bis 8; b: 2 bis 12; c: 1 oder 2; m: 2 bis 6, und

$R_5$ bis $R_9$ unabhängig voneinander H, $C_1$-$C_8$ -Alkyl, $C_3$-$C_8$-Alkenyl, $C_5$-$C_8$-Cycloalkyl, $C_7$-$C_9$-Phenylalkyl, HO-$C_2H_4$, HO-$C_3H_6$, AYN-$(CHR_1)_m$, oder $R_6$ und $R_7$ bzw. $R_8$ und $R_9$ jeweils zusammen gegebenenfalls durch O, NH oder N-$CH_3$ unterbrochenes $C_2$-$C_5$-Alkylen bilden.

**2.** Verbindungen gemäß Anspruch 1, worin

A und Y unabhängig voneinander $C_1$-$C_8$ -Alkyl oder $C_5$-$C_8$-Cycloalkyl sind oder zusammen gegebenenfalls durch O, NH oder N-$CH_3$ unterbrochenes $C_2$-$C_5$-Alkylen bilden,

$R_1$: H,

$R_2$ : $C_1$-$C_8$-Alkyl, $NH_2$ ,

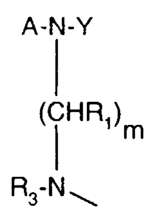 , 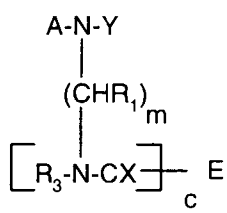

oder Phenyl,

$R_3$: H, $C_1$-$C_4$-Alkyl,

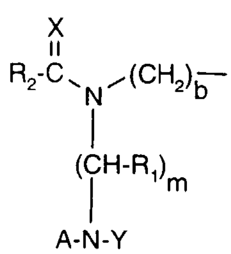 oder 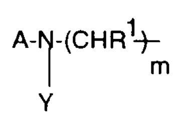 ,

E : $(CH_2)_a$, -CH=CH-

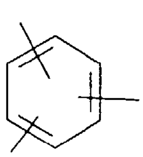 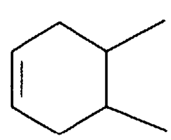 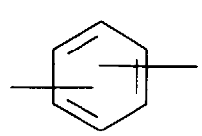

oder -NH $(CH_2)_bNH$-;

X: O, a: 0 bis 4, b: 2 bis 6, c: 1 oder 2 und

m: 2 oder 3 sind, und

$R_5$ bis $R_9$ unabhängig voneinander H, $C_1$-$C_8$ -Alkyl, $C_5$-$C_8$-Cycloalkyl, HO-$C_2H_4$-, HO-$C_3H_6$-F oder

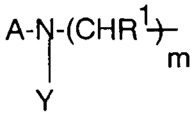 ,

sind, oder

$R_6$ und $R_7$ bzw. $R_8$ und $R_9$ zusammen jeweils gegebenenfalls durch O, NH oder N-$CH_3$ unterbrochenes $C_2$-$C_5$Alkylen bilden.

3. Verbindungen gemäß Anspruch 1, worin A und Y gleich und $C_1$-$C_8$ -Alkyl oder Cyclohexyl sind,

$R_1$: H,

$R_2$:

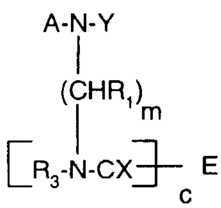

$R_3$: H, $C_1$-$C_8$-Alkyl,

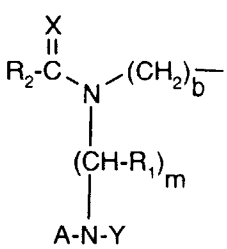 oder 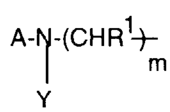 ,

E : $(CH_2)_a$, oder -NH $(CH_2)_bNH$-;

X: O ;

a: 0 bis 4; b: 2 bis 6; c: 1 oder 2; m: 2 oder 3; und

$R_5$ bis $R_9$ unabhängig voneinander H, $C_1$-$C_4$ -Alkyl,

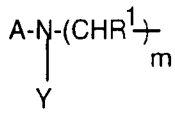 ,

sind.

**4.** Stabilisatorkombination enthaltend

**A)** mindestens eine organischen Zinkverbindung mit einer Zn-O- oder Zn-S-Bindung, und

**B)** mindestens eine Verbindung der Formel I oder II gemäß Anspruch 1.

**5.** Stabilisatorkombination gemäß Anspruch 4, enthaltend zusätzlich Alkali-und/oder Erdalkalicarboxylate.

**6.** Stabilisatorkombination gemäß Anspruch 4, enthaltend zusätzlich Erdalkali-und/oder Aluminumcarboxylate.

**7.** Stabilisatorkombination gemäß Anspruch 4, enthaltend zusätzlich epoxidierte Fettsäureester.

**8.** Stabilisatorkombination gemäß Anspruch 4, enthaltend zusätzlich Phosphite.

**9.** Stabilisatorkombination gemäß Anspruch 4, enthaltend zusätzlich mindestens eine Verbindung aus den Gruppen der Perchlorat-Verbindungen,
Glycidylverbindungen, 1,3-Diketone und 1,3-Diketoester, Dihydropyridine und Polydihydropyridine, Polyole, Disaccharidalkohole, sterisch gehinderten Amine (2.2.6.6-Tetraalkylpiperidinverbindungen), Zeolithe, Hydrotalcite, sowie der Alkalialumocarbonate (Dawsonite).

**10.** Stabilisatorkombination gemäß Anspruch 4, enthaltend zusätzlich mindestens eine Verbindung aus den Gruppen der Antioxidantien, Lichtschutzmittel, UV-Absorber, Weichmacher, Füllstoffe, Pigmente und Gleitmittel.

**11.** Zusammensetzung enthaltend ein organisches Material und eine Stabilisatorkombination gemäß Anspruch 4.

**12.** Zusammensetzung gemäß Anspruch 11, worin das organische Material ein chlorhaltiges Polymer ist.

**13.** Verfahren zur Stabilisierung chlorhaltiger Polymere, dadurch gekennzeichnet, daß in die chlorhaltigen Polymere eine Stabilisatorkombination gemäß Anspruch 4 eingearbeitet wird.